# EUROPÄISCHE PATENTSCHRIFT

(11) **EP 0 575 908 B1**
(45) Veröffentlichungstag und Bekanntmachung des Hinweises auf die Patenterteilung: **15.03.2000**
(21) Anmeldenummer: 93109771.1
(22) Anmeldetag: 18.06.1993
(51) Int. Cl.: C12P 19/02, C12R 1/385

(54) **Pseudomonas aeruginosa und seine Verwendung zur Herstellung von L-Rhamnose**
Pseudomonas aeruginosa and its use in the preparation of L-rhamnose
Pseudomonas aeruginosa et son utilisation pour la préparation du L-rhamnose

(30) Priorität: 25.06.1992 DE 4220437; 31.07.1992 DE 4225283
(43) Veröffentlichungstag der Anmeldung: 29.12.1993
(73) Patentinhaber: Aventis Research & Technologies GmbH & Co. KG, 65926 Frankfurt am Main (DE)
(72) Erfinder: Giani, Carlo, Dr., D-6000 Frankfurt/Main 70 (DE); Wullbrandt, Dieter, Dr., D-6238 Hofheim/Ts. (DE); Rothert, Reinhardt, W-6272 Niedernhausen (DE); Meiwes, Johannes, Dr., D-6270 Idstein (DE)

(56) Entgegenhaltungen:
- EP-A- 0 153 634
- EP-A- 0 282 942
- GB-A- 2 194 247
- ZEITSCHRIFT FÜR NATURFORSCHUNG, Bd.40c, Nr.1,2, 1985, TÜBINGEN DE Seiten 61 - 67 SYLDATK C et al 'PRODUCTION OF 4 INTERFACIAL ACTIVE RHAMNOLIPIDS FROM N ALKANES OR GLYCEROL BY RESTING CELLS OF PSEUDOMONAS-SP DSM-2874'
- APPLIED AND ENVIRONMENTAL MICROBIOLOGY, Bd.51, Nr.5, 1986, WASHINGTON D C Seiten 985 - 989 REILING H E et al 'PILOT PLANT PRODUCTION OF RHAMNOLIPID BIOSURFACTANT BY PSEUDOMONAS-AERUGINOSA'
- DATABASE BIOTECHNOLOGY DERWENT PUBLICATIONS, LONDON, GB 91-08513 1991, SHABTAI Y 'Isolation and characterization of a lipolytic bacterium capable of growing in a low-water-content oil-water emulsion'

## Beschreibung

Der Desoxyzucker L-Rhamnose (6-Desoxy-L-Mannose) eignet sich als chiraler Baustein sehr gut zur Herstellung verschiedener organischer Verbindungen. L-Rhamnose oder deren Derivate finden immer breitere Anwendung bei der Synthese von pharmazeutischen Erzeugnissen und Pflanzenschutzmitteln, wie auch im Bereich der Cytologie pflanzlicher und tierischer Zellen, der Mikrobiologie, Immunbiologie und Aromaherstellung. So läßt sich z. B. mit L-Rhamnose als Ausgangsverbindung 2,5-Dimethyl-4-hydroxy-2,3-dihydrofuran-3-on (Furaneol®) herstellen, das wiederum als Bestandteil verschiedener Aromastoffe in der Nahrungsmittel- und Duftstoffindustrie dient.

Der Zucker L-Rhamnose ist auf chemischem Weg nur sehr schwer zugänglich. Er läßt sich aber aus verschiedenen, natürlichen Quellen extraktiv nach saurer oder enzymatischer Hydrolyse gewinnen, so z. B. aus den flavonoiden Glycosiden Hesperidin, Rutin, Naringin, Quercitrin oder z. B. aus Gummi arabicum oder Meeresalgen. [Biotechnology and Bioengineering, Vol. 33, S. 365 (1989), R. J. Linhardt et al.; EP-A-0 317 033; JPA 62293; U.S. Patent Nr. 5,077,206, Cheetham et al.]. Nachteilig für die Verfahren der sauren Hydrolyse wirken sich die prozeßintensiven Isolierungsschritte für L-Rhamnose, teilweise unter Verwendung organischer Lösungsmittel, ferner die bei der Aufarbeitung anfallenden aromatischen, potentiell toxischen Abfallprodukte und die in der Zusammensetzung schwankenden, vom jahreszeitlichen Rhythmus abhängigen Inhaltsstoffe der natürlichen Quellen aus.

Im U.S. Patent Nr. 5,077,206 ist ein Verfahren zur Herstellung von L-Rhamnose aus Pflanzenmaterial unter Verwendung mehrerer Enzyme und nachfolgend mehrerer Reinigungsschritte beansprucht. Nachteilig an diesem Verfahren ist die schwierige Abtrennung der L-Rhamnose von den aus dem Pflanzenmaterial stammenden Zuckern, insbesondere von Glukose, aufgrund der starken chemisch strukturellen Ähnlichkeiten der Zucker (beinhaltet auch das Molekulargewicht).

Fermentativ läßt sich L-Rhamnose auch in Form rhamnoseenthaltender Heteropolysaccharide mit Hilfe von Bakterien verschiedener Gattungen wie z.B. Alcaligenes, Acinetobacter, Klebsiella, Streptococcus oder Lactobacillus herstellen. [Enzyme Microb. Technol., Vol. 10, S. 198 (1988), M. Graber et al.; J. Amer. Chem. Soc., Vol. 71, S. 4124 (1945), F.G. Jarvis und M. J. Johnson; J. Bacteriol, Vol. 68, S. 645 (1954), G. Hauser and M. L. Karnovsky].

Nachteilig bei diesen Verfahren sind die üblicherweise viskositätsbedingt geringen Ausbeuten und die nach hydrolytischer Spaltung des Heteropolysaccharides notwendige, schwierige Trennung der L-Rhamnose (Gründe: s.o.) aus einem Gemisch verschiedener Zucker. In einer weiteren Literaturstelle wird ein Rhamnose enthaltendes Heteropolysaccharid beschrieben, das fermentativ mit einem Bakterium der Gattung Klebsiella hergestellt wird. Die Rhamnoseausbeuten betragen, bezogen auf die Kulturlösung, ca. 17 g/l [Commission Of The European Communities, ECLAIR-Program, Contract No. AGRE-0011-C., Report No. 2 (final report) 1991].

Eine weitere biogene Quelle für 6-Desoxyzucker stellen die Glycolipide dar, die fermentativ hergestellt werden können [Microbiological Sciences Vol. 3, No. 5, S. 145, (1986), D. G. Cooper; Surfactant Sci. Series, Vol. 25, S. 89 (1987), Christoph Syldatk and Fritz Wagner; Biotechnology and Bioengineering, Vol. 33, S. 365 (1989), R. J. Linhardt et al.; US-Patent 4 933 281, Daniels et al.; US-Patent 4 814 272, Wagner et al.].

GB-A-2 194 247 beschreibt ein Verfahren zur Herstellung von L-Phenylalanin unter Verwendung von Mikroorganismen, unter anderem der Gattung Pseudomonas, wobei die Mikororganismen mutagenen Behandlungsschritten und Selektionsschritten unterzogen worden sind. Seit langem ist bekannt, daß Rhamnolipide von dem Bakterium Pseudomonas aeruginosa gebildet werden [J. Amer. Chem. Soc., Vol. 71, S. 4124 (1949), F. G. Jarvis et al.; J. Bacteriol., Vol. 68, S. 645 (1954) George Hauser und Manfred L. Karnovsky]. Zahlreiche Veröffentlichungen und Patente befassen sich mit der fermentativen Gewinnung von Rhamnolipiden durch Pseudomonas aeruginosa. [Applied and Environmental Microbiology, Vol. 51, No. 5, S. 985 (1986), H.E. Reiling et al.; J. Chem. Techn. Biotechnol. Vol. 45, S. 249 (1989), K. Venkata Ramana et al.; US-Patent 4 933 281, Daniels et al.; Deutsche Offenlegungsschrift 2 150 375, 1972; US-Patent 4 814 272, Wagner et al.]

In der Kulturlösung von Pseudomonas aeruginosa kommen hauptsächlich 4 Rhamnolipide (RL1 - RL4, siehe Abb. 1) vor, die aus 1 oder 2 L (+)-Rhamnose-Einheiten und ein oder zwei β-Hydroxydecansäuren bestehen [Z. Naturforsch. 40 c, S. 61 (1985), C. Syldatk et al.].

### Abbildung 1: Rhamnolipide von Pseudomonas aeruginosa

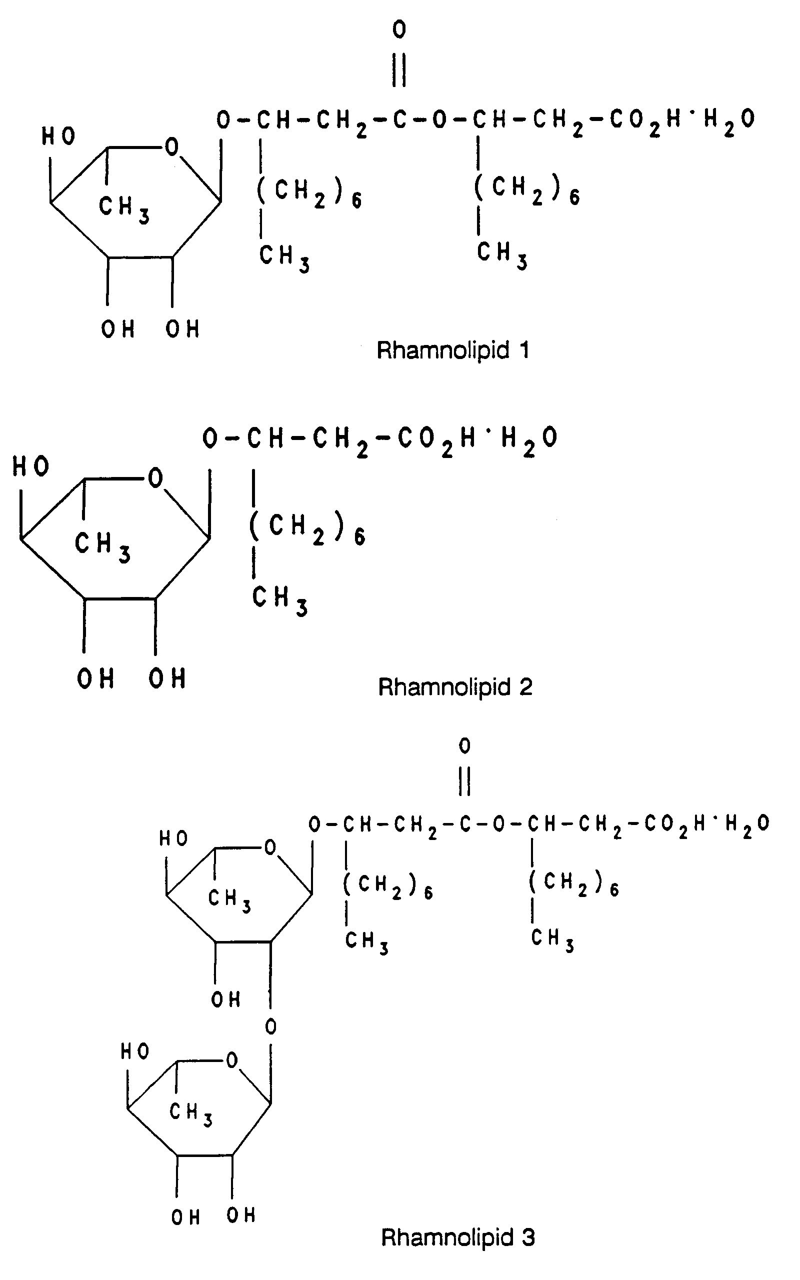

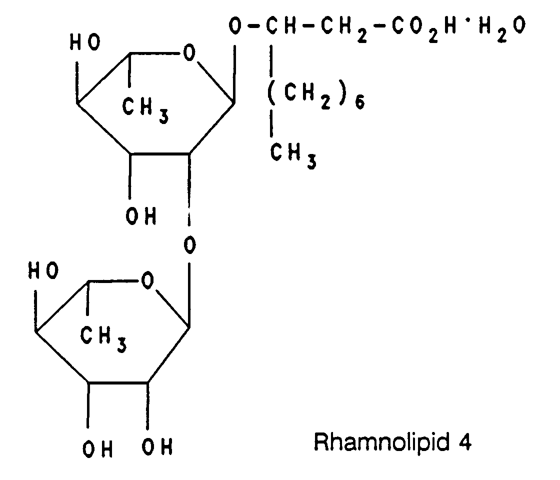

Eine neue Veröffentlichung zeigt, daß Pseudomonas aeruginosa in der Lage ist, weitere Rhamnolipide zu bilden [Biochimica et Biophysica Acta, Vol. 1045, S. 189, (1990) N. B. Rendell et al.]. Diese können jedoch mengenmäßig nicht mit den Rhamnolipiden 1 - 4 konkurrieren. Sie setzen sich neben L-Rhamnose und β-Hydroxydecansäure noch aus 3-Hydroxyoctansäure, 3-Hydroxydodecansäure oder 3-Hydroxydodecan-5-en-säure zusammen.

Neben Glucose, Glycerin, n-Alkanen, Fettalkoholen und Fettsäuren eignen sich auch pflanzliche Öle wie Sojaöl oder Olivenöl als C-Quellen für eine Rhamnolipid-Produktion [Z. Naturforsch. 40 c, S. 61 (1985), C. Syldatk et al., Surfactant Sci. Series, Vol. 25, S. 89 (1987), C. Syldatk et al.; Applied and Environmental Microbiology, Vol. 51, No. 5, S. 985 (1986), H. E. Reiling et al.; Biotechnology and Bioengineering, Vol. 33, S. 365 (1989), R. J. Linhardt et al.; Agr. Biol. Chem.; Vol. 35, No. 5, S. 686 (1971), H. Hisatsuka et al., US-Patent 4 814 272 Wagner et al.]. Das Verhältnis der Rhamnolipide zueinander und ihre Ausbeuten hängen weitgehend von den Kulturbedingungen ab [Z. Naturforsch. 40 c, S. 61 (1985), C. Syldatk et al.; Tenside Surf. Det. 27, 5, S. 302 (1990), J. L. Parra et al.].

So zeigten Parra et al., daß vorwiegend RL1 und RL3 bei der Verwendung von Olivenöl als C-Quelle gebildet werden.

Die bislang fermentativ maximal erzielten Ausbeuten an Rhamnolipiden sind im US-Patent 4 933 281 (Daniels et al.) äquivalent zu EP-A-0 282 942, beschrieben. Aus den Ansprüchen geht hervor, daß bei Verwendung von Maisöl (Corn-oil) als C-Quelle nach der Fermentation eines Pseudomonas aeruginosa Stammes die Rhamnolipide in einer Konzentration von 30 - 50 g/l aus dem Kulturmedium isoliert werden. Die Isolierung erfolgt, um aufgereinigte Rhamnolipide zu erhalten. In der nachfolgenden Hydrolyse der isolierten Rhamnolipide entstehen als Reaktionsprodukte L-Rhamnose und Hydroxydecansäure.

Die in dem Patent enthaltenden Beispiele, insbesondere Beispiel 3, machen deutlich, daß die in den Ansprüchen angegebene Rhamnolipidkonzentration von 30 - 50 g/l sich auf die in der Kulturlösung vorhandenen Konzentrationen beziehen.

Es wurde nun überraschend ein Verfahren zur Herstellung von L-Rhamnose durch Fermentation von Pseudomonas aeruginosa gefunden, mit dessen Hilfe in der Fermentationslösung Rhamnolipid-Konzentrationen von 70 - 120 g/l erzielt werden. Die Herstellung der L-Rhamnose erfolgt eine aufwendige Abtrennung der Zellmasse aus der Kulturlösung und ohne eine Isolierung der Rhamnolipide vor der Hydrolyse.

Die Erfindung betrifft somit:
1. Ein Verfahren zur Herstellung von L-Rhamnose, das dadurch gekennzeichnet ist, daß durch Fermentation von Pseudomonas aeruginosa Rhamnolipide synthetisiert und die Rhamnolipide ohne vorherige Isolierung zu L-Rhamnose hydrolysiert werden.
2. Pseudomonas aeruginosa DSM 7107 und DSM 7108 mit der Fähigkeit, Rhamnolipide in einer Konzentration von 70 - 120 g/l Kulturlösung zu synthetisieren.

Im folgenden wird die Erfindung detailliert beschrieben. Ferner wird sie durch den Inhalt der Ansprüche bestimmt.

Die Fermentation kann im Labormaßstab (Fermentationsmenge im Literbereich) ebenso wie im industriellen Maßstab (z.B. im 20 - 50 m³ Maßstab) durchgeführt werden.

Prozentangaben beziehen sich, wenn nicht anders angegeben, auf das Gewicht.

Als Mikroorganismen können alle Bakterienstämme fermentiert werden, die Rhamnolipide in den Kulturüberstand sezernieren.

Die Mikroorganismen werden mit Hilfe von Anreicherungskulturen aus ihrer natürlichen Umgebung isoliert. Diese Vorgehensweise ist dem Fachmann bekannt. Kurz zusammengefaßt:

Die Anreicherungsbedingungen sind diejenigen, unter denen sich ein Organismus bei Konkurrenz durchsetzt. Für öl- und fettliebende Mikroorganismen, wie z. B. Pseudomonas aeruginosa, werden Minimalmedien mit Ölen, Fetten und/oder Kohlenwasserstoffen als Kohlenstoffquellen verwendet. Man stellt diese Umweltbedingungen her und impft mit einer gemischten Population, wie sie in der natürlichen Umgebung vorliegt. In einer solchen Anreicherungsnährlösung setzen sich die gewünschten Bakterienstämme durch und überwachsen alle Begleitorganismen. Durch mehrfache Übertragung auf die gleiche Nährlösung und Verteilen auf einen festen Nährboden läßt sich der angereicherte Stamm leicht isolieren. Eine häufige, nach kurzen Intervallen erfolgende "Flüssig-flüssig-Überimpfung" beugt dem Wachstum von Begleitorganismen vor, die die Ausscheidungs- oder gar Autolyseprodukte der primär begünstigten Zellen nutzen würden (Allg. Mikrobiologie von H. G. Schlegel, 6. Auflage, S. 182, G. Thieme Verlag Stuttgart, New York).

Vorzugsweise werden Bakterien verwendet, die aus einer Anreicherungskultur einer Wasserprobe isoliert wurden. Insbesondere wird die Wasserprobe eines öl-und fettverarbeitenden Betriebs verwendet.

Die verwendete Wasserprobe stammt aus werkseigenem Wasser. Der aus dieser Wasserprobe isolierte Stamm wurde von der Deutschen Sammlung von Mikroorganismen und Zellkulturen GmbH, Mascheroder Weg 1 B, W-3300 Braunschweig, Deutschland als Pseudomonas aeruginosa bestimmt.

Die Zellen von Pseudomonas aeruginosa sind stäbchenförmig, haben einen Durchmesser von ca. 0,6 - 0,8 µm, sind ca. 1,5 - 3,0 µm lang und sind beweglich.

Nach der Isolierung erfolgt die Mutagenisierung von Pseudomonas aeruginosa. Die Mutagenese von Pseudomonas aeruginosa wird in an sich bekannter Weise mit Hilfe des chemischen Mutagens N-Methyl-nitro-N'-nitroso-guanidin (MNNG) durchgeführt.

Nach der Mutagenese von Pseudomonas aeruginosa wird mit Hilfe der Durchflußcytometrie eine Zellvereinzelung zur Isolierung von Produzentenstämmen vorgenommen. Dabei wurden die optischen Eigenschaften der suspendierten, mit MNNG behandelten Zellen im Durchfluß gemessen. Zellen typischer oder unterschiedlicher Größe und Form wurden so automatisch auf unterschiedliche Nährböden vereinzelt.

Verwendet wird ein handelsüblicher Cytofluometer mit folgender Ausstattung: Argon-Laser (Wellenlänge: 488 m, Leistung: 20 mW); Meßvorrichtung für die opt. Signale Vorwärtsstreulicht und 90°-Streulicht zur Beurteilung der Zellgröße und -form; automatische Sortiereinheit.

Die Sortierung von Einzelzellen wurde auf 2 unterschiedlichen Nährböden, einem dem Fachmann bekannten Glycerin-Minimalnährboden und einem Sojaöl-Minimalnährboden, vorgenommen.

Nach einer Bebrütung der Nährböden bei 37 °C wurden die zu Klonen ausgewachsenen Einzelzellen auf ihre Produktivität an Rhamnolipiden in Schüttelkolben und Kleinfermentern überprüft.

Auf diese Art konnten 2 Hochleistungsmutanten von Pseudomonas aeruginosa isoliert werden. Diese Mutanten werden besonders bevorzugt zur Fermentation und damit zur Herstellung von L-Rhamnose eingesetzt.

Sie sind, ebenso wie der nicht-mutagenisierter Pseudomonas aeruginosa, stäbchenförmig, haben einen Durchmesser von ca. 0,6 - 0,8 µm, sind ca. 1,5 - 3,0 µm lang und sind beweglich.

Die Hochleistungsmutanten von Pseudomonas aeruginosa wurden unter der Bezeichnung DSM 7107 und DSM 7108 bei der Deutschen Sammlung von Mikroorganismen und Zellkulturen GmbH, Mascheroder Weg 1 B, W-3300 Braunschweig, Deutschland, am 16. Juni 1992 nach den Regeln des Budapester Vertrages hinterlegt.

Solche Mutanten können auch in anderer bekannter Weise durch physikalische Mittel, beispielsweise Bestrahlung mit Ultraviolett- oder Röntgenstrahlen oder mit Hilfe von anderen chemischen Mutagenen, wie beispielsweise Ethylmethansulfonat (EMS) oder 2-Hydroxy-4-methoxybenzophenon (MOB) erzeugt werden.

Die im folgenden beschriebene Vorgehensweise gilt für alle Pseudomonas aeruginosa Isolate.

Pseudomonas aeruginosa wird in einem Medium mit Pflanzenölen als Kohlenstoffquelle fermentiert. Beispiele für Pflanzenöle sind Raps-, Oliven-, Mais- und Sonnenblumenkernöl. Das Medium muß außer Pflanzenöl noch eine oder mehrere Stickstoffquellen, Sulfat- und Magnesiumionen sowie Kalium- und Chloridionen, eine oder mehrere Phosphorquellen und Spurenelemente enthalten. Als Pflanzenöl wird besonders Sojaöl in Konzentrationen von ca. 100 - 250 g/l Nährlösung verwendet, wobei Konzentrationen zwischen 125 g/l und 165 g/l zu bevorzugen sind.
Als Stickstoffquellen können dem Fachmann bekannte N-Quellen, wie z. B. (NH₄)₂SO₄ in Konzentrationen von 5 - 50 g/l Nährlösung verwendet werden, wobei vorzugsweise NaNO₃ in einer Konzentration von 15 g/l eingesetzt wird.

Zur Bereitstellung von Sulfat- und Magnesiumionen wird 0,01 - 2 g/l MgSO₄·7H₂O, vorzugsweise 0,5 g/l, eingesetzt. Die zur Produktion benötigten Kalium- und Chloridionen können durch den Einsatz von 0,1 bis 5 g/l KCl bereitgestellt werden, wobei 1 g/l zu bevorzugen ist.

Als Phosphorquelle und zur Pufferung des Mediums wird ein 0,001 bis 0,1 molarer Natriumphosphatpuffer eingesetzt. Vorzugsweise werden dazu ca. 6,5 g/l einer 75 %igen Phosphorsäure und ca. 8,9 g/l einer 33 %igen Natronlauge verwendet.

Eine FeCl₃ enthaltende Spurenelementelösung mit verschiedenen Metallsalzen, gelöst in einer wäßrigen Lösung von Natriumcitrat, wird nach der Sterilisation des Mediums entweder in 4 - 5facher Konzentration zugegeben oder vorzugsweise 4 - 5 mal in anfänglicher Konzentration zu unterschiedlichen Zeiten der Fermentationslösung zugesetzt.

Der pH-Wert der Nährlösung sollte bei Fermentationsstart zwischen pH 5,5 und 7,5, vorzugsweise bei pH 6,3 liegen und braucht im Laufe der Fermentation nicht geregelt zu werden.

Die Belüftung wird mit steriler Luft vorgenommen, die in die gerührte Fermentationslösung eingeblasen wird. Die Belüftungsraten variieren zwischen 0,02 und 0,5 VVM (Volumen Luft pro Volumen Fermentationslösung pro Minute) und sind abhängig von der Fermentergeometrie, der Rührergeometrie, des Energieeintrags, vor allem aber vom aktuellen Zustand der Fermentationslösung. Typischerweise werden in der Anwachsphase ca. 0,3 ± 0,05 VVM und in der Produktionsphase 0,1 ± 0,03 VVM eingestellt.

In Abhängigkeit vom Schäumungsverhalten der Fermentationslösung werden während der Fermentation ca. 5 - 15 ml/l Kulturlösung eines handelsüblichen Silikonantischaummittels zugegeben, beispielsweise Antischaummittel VP 1133 von der Fa. Wacker Chemie GmbH (München, Deutschland), welches im englischsprachigen Raum auch Silicon Antifoam Agent VP 1133 genannt wird.

Die Fermentationstemperatur liegt zwischen 20°C und 40°C, bevorzugt bei 30 - 35°C. Die Fermentationsdauer beträgt ca. 4 - 11 Tage, vorzugsweise 6 - 8 Tage. Die Pseudomonas aeruginosa Stämme können als Einzel- oder Mischkultur fermentiert werden. DSM 7107 und 7108 sind sowohl für die Fermentation im Labormaßstab wie im industriellen Maßstab sehr gut geeignet.

Unter den oben genannten Fermentationsbedingungen bilden die Mikroorganismen vorwiegend die Rhamnolipide 1 und 3 (Abb. 1) mit einer volumentrischen Gesamtproduktivität an Rhamnolipiden von ca. 70 - 120 g/l Kulturlösung. Dabei werden Konzentrationen an L-Rhamnose von 30 - 50 g/l erzielt.

Die Gewinnung der L-Rhamnose erfolgt durch direkte Hydrolyse der Rhamnolipide, d. h. ohne eine Abtrennung der Zellmasse und ohne eine Isolierung der Rhamnolipide vor ihrer Hydrolyse zu L-Rhamnose.

Außer den Rhamnolipiden befinden sich in der Kulturlösung noch unverdautes Pflanzenöl, hauptsächlich Sojaöl, tote Zellen, sämtliche, nicht von den Mikroorganismen verbrauchte Kulturmediumsbestandteile, Fettsäuren, Entschäumer gelöste Salze sowie weitere, nicht näher bestimmte bakterielle Stoffwechselprodukte.

Zur weiteren Aufarbeitung der Fermentationslösung werden die sie enthaltenen Bakterien zunächst einmal abgetötet, indem man den gesamten Fermenterinhalt auf 80 - 120°C, vorzugsweise auf 100°C erhitzt und diese Temperatur 15 - 90 Min., vorzugsweise 60 Min. lang beibehält.

Die weiteren Verfahrensschritte der Aufarbeitung der abgekühlten Kulturbrühe, die im wesentlichen eine wäßrige Emulsion darstellt, werden in der Offenlegungsschrift WO 92/05183 (PCT-EP 91-01756, 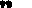 Verfahren zur Herstellung gereinigter Glycolipide durch Membrantrennverfahren") und der Offenlegungsschrift WO 92/05182 (PCT-EP 91-01426, 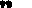 Verfahren zur Herstellung von L-Rhamnose aus Rhamnolipiden") beschrieben.

Die Aufarbeitungsschritte zur Isolierung der Rhamnose in logischer Reihenfolge sind:
1. Ansäuern der hitzeinaktivierten Kulturlösung.
2. Aufkonzentrieren durch Ultrafiltration an Membranen mit einer Trenngrenze von 30.000 bis 300.000 Dalton.
3. Entsalzen durch Diafiltration an der gleichen Membran.
4. Hydrolyse des entsalzten Konzentrates bei einem pH-Wert von 0 - 3 und einer Temperatur von 120 - 150 °C.
5. Abtrennen der wäßrigen, rhamnosehaltige Phase von der lipiden Phase.
7. Reinigung der wäßrigen Phase bei pH 3 - 8 durch Behandlung mit Entfärbungsmitteln wie Aktivkohle oder Bentonit oder durch Ionenaustauscherchromatographie.
8. Kristallisation der L-Rhamnose aus der eingeengten, wäßrigen Phase.

Das Ansäuern der hitzeinaktivierten Kulturlösung erfolgt mit Säuren, insbesondere entweder mit H₂SO₄, wenn die kontinuierliche Hydrolyse der Rhamnolipide zu L-Rhamnose durchgeführt wird oder im Falle der "Batchhydrolyse" insbesondere mit HCl oder H₂SO₄.

Unter diesen sauren Bedingungen werden Glykolipide, die normalerweise die Ultrafiltrationsmembran passieren könnten, zurückgehalten.

Im Anschluß an die Ultrafiltration werden durch Diafiltration an der gleichen Membran die Salze durch permanente Zugabe von Wasser und Abnahme von Filtrat teilweise herausgewaschen. Die Salzkonzentration wird durch die Leitfähigkeitsmessungen während der Filtration bestimmt. Die Leitfähigkeitsmessung wird mit handelsüblichen Elektroden durchgeführt.

Als Resultat dieser Vorgehensweise (Aufkonzentrierung und Waschen) entsteht eine wäßrige, 2 - 3fach aufkonzentrierte Lösung, die im wesentlichen folgende Bestandteile beinhaltet: nicht-metabolisierte Fettsäuren und Sojaölreste, Salze (Leitfähigkeit auf ca. 20 % reduziert), tote Zellen, Entschäumer, Rhamnolipide und andere bakterielle, höhermolekulare Stoffwechselprodukte. Die Rhamnolipide werden bei dieser Vorgehensweise vollständig zurückgehalten.

Die chemische Hydrolyse der Rhamnolipide wird ohne deren vorherige Isolierung direkt in der Kulturlösung, welche wie oben beschrieben behandelt worden ist, durchgeführt.

Die chemische Hydrolyse findet im homogenisierten Zustand, d. h. unter Rühren statt. Im Anschluß an die Hydrolyse werden die wäßrige und lipidhaltige Phase nach dem Fachmann bekannten Methoden getrennt. Die Isolierung der L-Rhamnose erfolgt aus der wäßrigen Phase.

Wurde im ersten Schritt (Ansäuerungsschritt) H₂SO₄ eingesetzt, wird die wäßrige Phase mit Ca(OH)₂ oder CaCO₃ zur Entfernung von H₂SO₄ versetzt. Abschließend erfolgt die Aufreinigung der wäßrigen Phase bei pH 3 - 8 durch Behandlung mit Entfärbungsmitteln oder durch Ionenaustauscherchromatographie. Die während der Hydrolyse in geringen Mengen entstandene Hydroxydecansäure wird nicht isoliert.

### 3. Beispiele

### 3.1. Beispiel 1:

### Gewinnung der Pseudomonas aeruginosa Isolate:

### a) Anreicherungskultur

Die Mikroorganismen werden aus werkseigenen Wasserproben gewonnen. Hierfür werden ca. 10 ml Abwasserprobe in einem 500 ml Erlenmeyerkolben mit 200 ml Anreicherungsmedium (Mineralsalzmedium s. u.) bei 37 °C 3 Tage lang auf der Schüttelmaschine bebrütet.

| Mineralsalzmedium: | |
|---|---|
| 15 g/l | Olivenöl |
| 10 g/l | NaNO₃ |
| 0,1 g/l | CaCl₂·2H₂O |
| 0,4 g/l | MgSO₄·7H₂O |
| 6,8 g/l | KH₂PO₄ |
| 8,7 g/l | K₂HPO₄·3H₂O |
| 5 ml/l | Spurensalze |
| deionisiertes Wasser | |

| Spurensalze: | |
|---|---|
| 1 g/l | Eisen(III)citrat |
| 0,2 g/l | MnSO₄ |
| 0,1 g/l | ZnCl₂ |
| 0,025 g/l | CuSO₄·5H₂O |
| 0,02 g/l | Natriumtetraborat |
| 0,01 g/l | Natriummolybdat |
| 0,004 g/l | CoCl₂ |
| deionisiertes Wasser | |

2 ml der 1. Anreicherungskultur werden in einem 2. Kolben mit dem gleichen Medium erneut wie oben beschrieben bebrütet.

Dieser Vorgang wird noch viermal wiederholt.

### b) Isolation

Die Kulturlösung der letzten Anreicherungskultur wird auf einer Agarplatte mit Vollmedium bestehend aus 10 g/l Glucose, 4 g/l Caseinpepton,
4 g/l Fleischextrakt, 0,5 g/l Hefeextrakt, 0,5 g/l Leberextrakt und 2,5 g/l NaCl, ausplattiert. Nach der Bebrütung bei 37 °C wird Pseudomonas aeruginosa als Klon isoliert.

### c) MNNG-Mutagenese

Der Klon aus Beispiel 1, Absatz b, wird 24 Std. im Vollmedium bei 37 °C kultiviert. Anschließend wird das Kulturmedium 1 : 10 mit frischem Vollmedium verdünnt. Die Zellen werden 2 Stunden bei 37 °C auf der Schüttelmaschine regeneriert.

Die durch Zentrifugation abgetrennten und im Tris-Maleinsäure-Puffer (pH 6) gewaschenen und wieder aufgenommenen Zellen werden mit MNNG (0,4 - 0,8 mg/ml) bei 37 °C 15 - 20 Min. lang auf dem Schüttler behandelt.

Nach dem wiederholten dreimaligen Waschen der Zellen in Tris-Maleinsäure-Puffer wird die mutagenisierte Zellsuspension im Vollmedium bei 37 °C 24 Std. lang kultiviert.
Mit Hilfe der Durchflußcytometrie wird anschließend eine Zellvereinzelung vorgenommen.

Die Sortierung der Einzelzellen wird auf folgendem Nährboden vorgenommen:

| Mininmal-Nährboden: | |
|---|---|
| 20 g/l | Glycerin (oder Sojaöl**) |
| 10 g/l | NaNO₃ |
| 1 g/l | KCl |
| 1 g/l | NaCl |
| 0,02 g/l | CaCl₂·2H₂O |
| 6,8 g/l | KH₂PO₄ |
| 8,7 g/l | K₂HPO₄ |
| 0,5 g/l | MgSO₄·7H₂O |
| 2 m/l | Spurenelemente-Lösung* |
| 20 g/l | Agar |
| deionisiertes Wasser pH-Einstellung auf pH 6,5 vor der Sterilisation | |

| | |
|---|---|
| * Zugabe nach der Sterilisation | |
| ** Homogenisierung des erhitzten Nährbodens kurz vor dem Gießen | |

| Spurenelementelösung: | |
|---|---|
| 1 g/l | Natriumcitrat·2H₂O |
| 0,14 g/l | FeCl₃·6H₂O |
| 0,7 g/l | ZnSO₄·7H₂O |
| 0,6 g/l | CoCl₂·6H₂O |
| 0,6 g/l | CuSO₄·5H₂O |
| 0,4 g/l | MnSO₄·5H₂O |
| deionisiertes Wasser | |

Die auf diese Weise gewonnenen Stämme werden für die im folgenden beschriebenen Fermentation eingesetzt.

### 3.2. Beispiel 2:

### Batch-Fermentation im Industriemaßstab zur Gewinnung von L-Rhamnose

a) Vorkultur
Eine erste Vorkultur des Stammes Pseudomonas aeruginosa DSM 7107 in 4 l Vorkultur-Nährlösung (Tab. 1) wird in Schüttelkolben hergestellt (2 l-Erlenmeyerkolben mit je 500 ml Nährlösung, 30 °C, 200 UpM, 20 h). Die gesamte 1. Vorkultur wird zum Beimpfen der 2. Vorkultur (350 l) verwendet.
Dazu wird in einem 450 l-Fermenter mit 350 l der komplexen Vorkultur-Nährlösung (Tabelle 1) der Stamm DSM 7107 aerob bei einer Belüftungsrate von 180 l Luft/Min. einer Rührergeschwindigkeit von 300 UpM, bei einer Temperatur von 28 °C 16 Stunden lang fermentiert.

**Tab. 1**

| Vorkultur - Nährlösung: |
|---|
| 10 g/l Glucose |
| 5 g/l Caseinpepton |
| 1 g/l Hefeextrakt |
| 0,5 g/l NaCl |
| Wasser |

Die gesamte 2. Vorkultur wird zur Beimpfung der Hauptkultur verwendet.
b) Hauptkultur
In einem Fermenter mit ca. 30m³ Nennvolumen werden 17 m³ der in Tabelle 2 angegebenen Nährlösung zubereitet:

**Tab. 2**

| Hauptkultur - Nährlösung: |
|---|
| 6,47 g/l 75%ige H₃PO₄ |
| ca. 8,94 g/l 33%ige NaOH |
| 0,5 g/l MgSO₄·7H₂O |
| 1 g/l KCl |
| 15 g/l NaNO₃ |
| 125 g/l Sojaöl |
| Wasser |

Dazu wird nach Vorlage der benötigten Wassermenge mit H₃PO₄ und NaOH ein pH-Wert von 6,8 eingestellt. Nach Zugabe der restlichen Nährlösungsbestandteile wird mit H₂SO₄ der pH-Wert auf pH 6,2 korrigiert.
Nach 45 minütiger Sterilisation hat sich ein pH-Wert von ca. 6,3 eingestellt. In einem separaten Behälter wird eine Lösung mit Spurenelementen (Tab. 3) sterilisiert. Dazu werden in 150 l deionisiertem Wasser folgende Substanzen gelöst und sterilisiert:

**Tab. 3**

| Spurenelementelösung: |
|---|
| 2 mg/l Natriumcitrat·2H₂O |
| 0,28 mg/l FeCl₃·6H₂O |
| 1,4 mg/l ZnSO₄·7H₂O |
| 1,2 mg/l CoCl₂·6H₂O |
| 1,2 mg/l CuSO₄·5H₂O |
| 0,8 mg/l MnSO₄·1H₂O |
| deionisiertes Wasser |
| Konzentrationsangaben beziehen sich auf 1 l Hauptkultur |

Diese Spurenelemente-Lösung wird vor der Beimpfung und 3 weitere Male nach ca. 20, 40 und 70 Stunden Fermentationsdauer dem Hauptfermenter unter sterilen Bedingungen zugegeben.
Als Inokulum wird der gesamte Inhalt des Vorfermenters (350 l) verwendet. Die Fermentationstemperatur beträgt 30 °C. In den ersten 10 Fermentationsstunden wird mit 250 m³ Luft/Stunde belüftet, von der 10. bis zur 30. Stunde mit 400 m³/h und ab der 30. Stunde mit 100 - 75 m³/h.
Zur Schaumbekämpfung wird ein separat sterilisiertes Silikonantischaummittel VP 1133 (Fa. Wacker) verwendet, das in Abhängigkeit vom Schäumungsverhalten der Fermentationslösung unter Zuhilfenahme einer Schaumelektrode in den Fermenter portionsweise dosiert wird.
Als Rührorgan wird ein Radialrührer mit vier Turbinen, die einen Durchmesser von 1040 mm aufweisen, eingesetzt (Rührer Ø: Fermenter Ø = 0,4 : 1). Die Drehzahl beträgt in den ersten 10 Fermentationsstunden 50 UpM, ab der 10. Stunde 75 UpM.
Unter den oben genannten Fermentationsbedingungen lassen sich in 167 Std. Fermentationsdauer pro Liter Kulturlösung ca. 78 g Rhamnolipide und ein L-Rhamnosegehalt von ca. 32 g L-Rhamnose erzeugen.
Nach Fermentationsende wird im Fermenter die gesamte Fermentationslösung zur Abtötung des Produktionsstammes auf 100 °C erhitzt und bei dieser Temperatur 1 Stunde lang gerührt. Die weiteren Aufarbeitungsschritte bis zur kristallinen L-Rhamnose wird analog zu den Patentschriften PCT/EP 91-01756 und PCT/EP 91-01426 durchgeführt.

### 3.3 Beispiel 3:

### Fed-Batch-Fermentation im Industriemaßstab zur Gewinnung von L-Rhamnose

18,5 m³ Hauptkulturmedium mit der Zusammensetzung aus Beispiel 1 werden mit 350 l Vorkultur (ebenfalls in Beispiel 1 beschrieben) beimpft. Als Produktionsstamm wird der Stamm Pseudomonas aeruginosa DSM 7108 eingesetzt. Vor der Beimpfung und nach 20, 40, 70 und 120 Stunden Fermentationsdauer wird unter sterilen Bedingungen je eine Spurenelementelösung (siehe Beispiel 1) zugegeben.

Die Belüftungsraten werden folgendermaßen variiert: Bei Fermentationsstart 250 m³/h, nach 10 Fermentationsstunden 350 m³/h und nach 30 Fermentationsstunden in Abhängigkeit von der Intensität der Schaumbildung 100-130 m³/h Luft.

In den ersten 10 Fermentationsstunden wird mit 50 UpM gerührt, danach mit 75 UpM. Von der 72sten bis zur 109ten Fermentationsstunde werden kontinuierlich weitere 564 l Sojaöl zudosiert.
Die Schaumbekämpfung erfolgt in gleicher Weise wie in Beispiel 1 beschrieben wurde. Unter den genannten Fermentationsbedingungen werden in 9 Tagen in der Kulturlösung 95 g/l Rhamnolipide und ein Gehalt an L-Rhamnose von 39 - 40 g/l erzeugt. Die weitere Aufarbeitung der Kulturlösung erfolgt wie in Beispiel 1 beschrieben wurde.

### 3.4 Beispiel 4:

### Batch-Fermentation zur Gewinnung von L-Rhamnose in 300 l-Maßstab

In einem Fermenter von 450 l Nennvolumen werden 300 l Hauptkulturmedium (Zusammensetzung wie in Beispiel 1) sterilisiert.
Beimpft wird mit 4 l einer Schüttelkolben-Vorkultur (Medium aus Beispiel 1) des Stammes Pseudomonas aeruginosa DSM 7108.
Folgende Fermentationsbedingungen werden eingestellt: Die Fermentationstemperatur beträgt 30 °C. In den ersten Fermentationsstunden wird mit 300 UpM gerührt ab der 30. Stunde mit 400 UpM. Bis zur 10. Fermentationsstunde wird mit 80 l/min belüftet, ab der 10. Fermentationsstunde mit 120 l/min und ab der 30. Fermentationsstunde mit 30 l/min. Vor der Beimpfung und nach 20, 40 und 70 Fermentationsstunden werden die Spurenelemente aus Beispiel 1, jeweils in 4 l E-Wasser sterilisiert, zugegeben. Nach 112 Stunden Fermentationsdauer werden erneut 11,3 kg Sojaöl steril zugegeben. Entsprechend dem Schäumungsverhalten wird das Silikon-Antischaummittel VP 1133 (Fa. Wacker) bedarfsweise zudosiert. Unter den genannten Fermentationsbedingungen werden in 11 Tagen ca. 112 g Rhamnolipide pro Liter Kulturlösung und ca. 46 g/l L-Rhamnose gebildet.
Die Aufarbeitung der Kulturlösung erfolgt so wie in Beispiel 1 beschrieben wurde.

## Patentansprüche

1. Verfahren zur Herstellung von L-Rhamnose umfassend die Fermentation von Pseudomonas aeruginosa, um Rhamnolipide zu synthetisieren und die Hydrolyse der Rhamnolipide zu L-Rhamnose, dadurch gekennzeichnet, daß die von Pseudomonas aeruginosa abgegebenen und in der Kulturlösung enthaltenen Rhamnolipide ohne ihre vorherige Isolierung zu L-Rhamnose hydrolysiert werden.

2. Verfahren nach Anspruch 1, dadurch gekennzeichnet, daß Pseudomonas aeruginosa DSM 7107 und/oder DSM 7108 fermentiert werden.

3. Verfahren nach Anspruch 1 oder 2, dadurch gekennzeichnet, daß die Rhamnolipide zu L-Rhamnose in einer Konzentration von 30 - 50 g/l Kulturlösung hydrolysiert werden.

4. Verfahren nach einem der Ansprüche 1 bis 3, dadurch gekennzeichnet, daß als Kohlenstoffquelle ein oder mehrere Pflanzenöle, insbesondere Sojaöl, zum Kulturmedium hinzugegeben werden.

5. Verfahren nach einem der vorhergehenden Ansprüche, dadurch gekennzeichnet, daß die Fermentationsdauer 4 - 11 Tage beträgt.

6. Verfahren nach einem der vorhergehenden Ansprüche, dadurch gekennzeichnet, daß die Belüftungsrate zwischen 0,02 und 0,5 VVM liegt.

7. Pseudomonas aeruginosa DSM 7107 mit der Fähigkeit, Rhamnolipide in einer Konzentration von 70 - 120 g/l Kulturlösung zu synthetisieren.

8. Pseudomonas aeruginosa DSM 7108 mit der Fähigkeit, Rhamnolipide in einer Konzentration von 70 - 120 g/l Kulturlösung zu synthetisieren.

## Claims

1. A process for the preparation of L-rhamnose comprising the fermentation of Pseudomonas aeruginosa in order to synthesize rhamnolipids and the hydrolysis of the rhamnolipids to L-rhamnose, which comprises hydrolyzing the rhamnolipids generated by Pseudomonas aeruginosa and contained in the culture solution to L-rhamnose without prior isolation thereof.

2. The process as claimed in claim 1, wherein Pseudomonas aeruginosa DSM 7107 and/or DSM 7108 are fermented.

3. The process as claimed in claim 1 or 2, wherein the rhamnolipids are hydrolyzed to L-rhamnose in a concentration of 30 - 50 g/l of culture solution.

4. The process as claimed in one of claims 1 to 3, wherein one or more vegetable oils, in particular soybean oil, are added to the culture medium as a carbon source.

5. The process as claimed in one of the preceding claims, wherein the fermentation period is 4 - 11 days.

6. The process as claimed in one of the preceding claims, wherein the aeration rate is between 0.02 and 0.5 VVM.

7. Pseudomonas aeruginosa DSM 7107 having the ability to synthesize rhamnolipids in a concentration of 70 - 120 g/l of culture solution.

8. Pseudomonas aeruginosa DSM 7108 having the ability to synthesize rhamnolipids in a concentration of 70 - 120 g/l of culture solution.

## Revendications

1. Procédé de préparation de L-rhamnose comprenant la fermentation de *Pseudomonas aeruginosa* en vue d'une synthèse de rhamnolipides et l'hydrolyse des rhamnolipides en L-rhamnose, caractérisé en ce qu'on met en oeuvre l'hydrolyse sans isolement préalable des rhamnolipides sécrétés par *Pseudomonas aeruginosa* et contenus dans le bouillon de culture.

2. Procédé selon la revendication 1, caractérisé en ce qu on fermente *Pseudomonas aeruginosa* DSM 7107 et/ou DSM 7108.

3. Procédé selon la revendication 1 ou 2, caractérisé en ce qu'on hydrolyse les rhamnolipides en L-rhamnose en une concentration de 30 à 50 g/L de bouillon de culture.

4. Procédé selon l'une des revendications 1 à 3, caractérisé en ce qu'on ajoute au milieu de culture, en tant que source de carbone, une ou plusieurs huiles végétales, en particulier l'huile de soja.

5. Procédé selon l'une des revendications précédentes caractérisé en ce que la durée de fermentation est de 4 à 11 jours.

6. Procédé selon l'une des revendications précédentes caractérisé en ce que le taux d'aération est compris entre 0,02 et 0,5 VVM.

7. *Pseudomonas aeruginosa* DSM 7107 capable de synthétiser des rhamnolipides en une concentration de 70 à 120 g/L de bouillon de culture.

8. *Pseudomonas aeruginosa* DSM 7108 capable de synthétiser des rhamnolipides en une concentration de 70 à 120 g/L de bouillon de culture.
